# EUROPEAN PATENT APPLICATION

(11) **EP 2 402 057 A1**
(43) Veröffentlichungstag der Anmeldung: **04.01.2012**
(21) Anmeldenummer: 09851509.1
(22) Anmeldetag: 20.11.2009
(51) Int. Cl.: A61N 5/06

(54) **VERFAHREN ZUR ABGESETZTEN ÜBERTRAGUNG EINES ENERGIEINFORMATIONSSIGNALS VON BELEBTEN UND UNBELEBTEN OBJEKTEN UND ENERGIEINFORMATIONSSIGNALKONVERTIERVORRICHTUNG**

(71) Anmelder: Etory Ltd, Mahe (SC)
(72) Erfinder: GERMANOV, Evgeny Pavlovich, Moscow 129085 (RU); KUTUSHOV, Mikhail Vladimirovich, Moscow 125414 (RU); GRINSHTEIN, Mark, 42141 Natania (IL); SHRAYBMAN, Mikhail, Herzlia (IL)
(74) Vertreter: Jeck, Anton
(86) Internationale Anmeldenummer: PCT/RU2009/000638
(87) Internationale Veröffentlichungsnummer: WO 2011/062518

(57) **Zusammenfassung**

Das Verfahren umfasst die Übertragung der Energie-Informationssignale auf den ersten Zwischenträger mit nachfolgender Informationsübertragung zum Eingang der Empfangseinrichtung und Ableitung dieses Signals an der Empfangsseite, indem das Signal auf den zweiten Zwischenträger (Endträger) übertragen wird, um es bei beliebiger Anwendung bzw. im beliebigen Anwendungsgebiet benutzen zu können. Um die Fernübertragung des Energie-Informationssignals von lebendigen und leblosen Objekten sicherzustellen, wird das Energie-Informationssignal vom Ausgangsobjekt in das Informationswellenbündel umgewandelt und in den kohärenten Zustand gebracht, indem es über wenigstens einen zwischen zwei Polarisatoren angeordneten Kristall durchgelassen wird. Danach wird das umgewandelte Signal auf den ersten Zwischenträger aufgezeichnet. Dann wird das Signal in die Empfangs- und Sendeeinrichtung eingeleitet und über die drahtgebundenen und drahtlosen Verbindungsnetze (Verbindungsleitungen) zusammen mit dem elektromagnetischen Signal übertragen. Danach wird das empfangene Signal auf den zweiten Träger gespeichert. Das dem Ausgangsobjekt inhärente (eigene) Energie-Informationssignal wird vom zweiten Träger abgelesen. Das Ablesen des dem Ausgangsobjekt inhärenten Energie-Informationssignals vom zweiten Träger wird unter Einsatz des biophysikalischen Geräts für Bioresonaztherapie oder vegetativen Resonanztest ausgeführt. Der Wandler enthält Kontakt 1, Becher (Behälter) 2 für die Aufnahme des Objekts zur Einwirkung (Beanspruchung) durch das superschwache Energie-Informationssignal, obere Grundplatte 3, die erste Stütze 4, Folienhalter 5, obere Polarisationsfolie 6 (Polarisator), Quarzplatte 7, Quarzhalter 8, den zweiten Folienhalter 9, die zweite Stütze 10, Justiermitnehmer 11, untere Polarisationsfolie 12 (Polarisator), unteren Ring 13 für Folienhalter 12, untere Grundplatte 14, die dritte Stütze 15, Dämpfer 16 und Auflagefläche für die Signalquelle. Es wird eine wirksame Fernübertragung der Energie-Informationssignale sichergestellt, das gesamte technische Verfahren wird vereinfacht. Bei der Anwendung der Erfindung in der Medizin wird die Dauer von Prozeduren gekürzt, die Funktionsmöglichkeiten der Diagnosestellung bei verschiedenen Pathologien und der Bestimmung von erfolgsreichen Behandlungsplänen werden erweitert.

## Beschreibung

Die Erfindung bezieht sich auf die Anwendung von einer neuen physikalischen Erscheinung. Sie kann in beliebigen technischen Gebieten und insbesondere in Medizin und Medizintechnik, in der Pharma-, Lebensmittel- und Chemieindustrie im Bereich Ökologie angewendet werden. Die Erfindung wird getrennt und /oder in Kombination mit dem vegetativen Resonanztest und /oder in Kombination mit Bioresonaztherapie ausgeführt.

Zur Zeit werden ausschließlich elektromagnetische Wellen zur Informationsübertragung bei großen Entfernungen verwendet (akustische Wellen sind nur für begrenzte Strecken geeignet). Normalerweise werden bei der Übertragung die VHF- (Meterwellen), UHF (Dezimeterwellen) und GHz-Wellenlängenbereiche benutzt. Die am häufigsten verwendeten Frequenzbereiche sind: 130 - 174 MHz; 350 - 450 MHz; 850 - 950 MHz und 1100 - 1300 MHz. Dabei werden für die Datenübertragung verschiedene Verfahren und Einrichtungen verwendet, die bei modernen Kommunikationsmitteln verbreitet sind. Bereits in der Anfangsphase der Datenübertragung wurden die digitalen Ansätze eingesetzt (z.B., Baudot-Kode). Wenn die Information unmittelbar für den Menschen und seinen Gebrauch vorgesehen ist, muss sie auf eine geeignete Weise umgewandelt werden. Das betrifft vor allem die Sprach- und Bildübertragung. Die Nachrichtenübertragungskanäle dienen in der Regel zur Übertragung von modulierten Signalen. Die Trägerfrequenz bei der Übertragung, z.B. bei Funkkanalsendung, überschreitet die dem Sprachsignal eigenen Frequenzen um einige Größenordnungen. Das Problem der Frequenzanpassung lässt sich durch Modulation lösen. Dabei muss jedoch beachtet werden, daß Modulation nicht nur in Funkkanälen verwendet wird. Die modernen digitalen Übertragungsverfahren sind ohne Modulation ebenfalls kaum denkbar.

Der Zusammenhang zwischen Kanalkapazität mit bestimmtem Durchlassbereich und dem Geräuschabstand wurde vom amerikanischen Ingenieur und Mathematiker Claude Shannon erforscht. Aus dem Shannons Leitsatz geht hervor: Bei Null- Geräuschpegel kann beliebig hohe Übertragungsgeschwindigkeit bei beliebig niedrigem Durchlaßbereich des Kanals erreicht werden. Solche Aussichten sollten für Fernsprechteilnehmer kaum freudig sein. Jedoch kann man sich die beliebige Menge der Signalpegel bei Rauschfreiheit auch innerhalb von 1 Volt gut vorstellen. Der Shannons Leitsatz klärt im Grunde genommen ab, wie der Rauschpegel den Grenzwert von V bei vorgegebener maximaler Signalamplitude begrenzt. Die genormten Verbindungsdrahtleitungen haben eine Dämpfung von 6 dB/km bei Frequenz von 800 Hz oder 10 dB/km bei Frequenz von 1600 Hz. Gleich von Anfang der Entwicklung von Fernsprechwesen an wurden die drahtgebundene Nachrichtentechnik und die Ausrüstung dafür aufgrund und abhängig von dem Vermögen des menschlichen Ohrs und Sprechapparats ausgelegt. Aus diesem Grund wiesen alle konventionellen Fernsprechsysteme den Durchlaßbereich von 3-3,5 kHz auf. Es gibt einen Zusammenhang zwischen Dämpfung und Frequenz des gesendeten Signals für eine Kupferleitung mit Querschnitt 0,5 mm. Die Kanalkapazität kann mit Hilfe von Breitbandeigenschaften und hohem Geräuschabstand vergrößert werden. Es gibt viele Geräuschquellen. Einer der wichtigsten davon ist Wärmerausch (N = kTB, wobei T für Temperatur, Grad Kelvin, B für Durchlaßbereich des Empfängers und k für Boltzmann-Konstante steht). In der Praxis wird wesentlich größerer Einfluß durch verschiedene eingestreute Störungen ausgeübt. Die Vergrößerung von Kanalkapazität im Netz wird durch solche Maßnahmen erreicht wie Kabelverkürzung (Kürzung der Strecken zwischen Netzknoten), Kabeltypwechsel, z.B. Austausch gegen einen Draht mit größerem Querschnitt, oder Anwendung von Lichtfaserkabel. Kupferleitungen werden wahrscheinlich in naher Zukunft durch Glasfaserhohlleiter ersetzt werden.

Bestimmter Effekt kann auch anhand des verbesserten Geräuschdämpfungssystems erreicht werden (neues wirksameres Modem).

Normalerweise werden zwei Fälle von Übersprechen betrachtet:
- Signalquelle und Empfänger am gleichen Kabelende (NEXT- near end crosstalk);
- Empfänger und Quelle an verschiedenen Kabelenden (FRXT - far end crosstalk).

Die aufgezeichneten Probleme bedürfen ihrer technischen Lösung. Vieles ist bereits gemacht worden, jedoch entstehen andere Probleme. Die modernen superschnellen Kommunikationssysteme decken neue Probleme und neue Lösungen auf. Was heute superschnell zu sein scheint (z.B. 6,4 Gb/Sek.), wird in einigen Jahren eine übliche Sache werden, so wie es mit Stammkanalzustand der Fall ist. Vor noch zehn Jahren waren 2 Mb/Sek. eine phantastische Geschwindigkeit. (2006 beträgt die Geschwindigkeit von Referenzkanälen im Netz der Russischen Akademie für Wissenschaften in Moskau bereits 10 Gb/Sek.). Man ist praktisch dicht an die theoretische Obergrenze der Übertragungsgeschwindigkeit gelangt. Diese Obergrenze wird durch die Polarisationszeit von Dielektrikum (10⁻¹³ Sek. - 10 THz).

Der Fortschritt der letzten Jahre in Sachen Kanalkapazitätserhöhung hängt in großem Maße mit der Entwicklung der digitalen Datenübertragungstechnologien zusammen. Hier müssen die Probleme der Synchronisation, der wirksamen Kodierung und zuverlässigen Übertragung. Je breiter Impuls desto höher ist seine Energie, desto besser ist der Geräuschabstand, desto niedriger ist auch der Grenzwert der Übertragungsgeschwindigkeit. Früher wurde jeder binären Stelle ein Impuls oder ein Unterschied in der Kode-Sequenz zugeordnet. Heute entsteht solcher Unterschied nur beim Wechsel von einer Nullen-Sequenz auf Einsen-Sequenz oder umgekehrt.

Das digitale Verfahren hat mehrere Vorteile gegenüber analoger Übertragung:
- Hohe Zuverlässigkeit. Unterschreitet das Geräusch den Eingangsschwellwert, so läßt sich sein Einfluß nicht fühlen, die erneute Kode-Sendung ist evtl. möglich.
- Keine Abhängigkeit von der Informationssignalquelle (Ton, Bild oder digitale Daten).
- Verschlüsselbarkeit, welche die Übertragungssicherheit erhöht.
- Zeitunabhängigkeit. Die Übertragung darf nicht unbedingt zum Zeitpunkt der Datenentstehung erfolgen sondern sobald der Kanal übertragungsbereit ist.

Um die Übertragungsgeschwindigkeit zu steigern, gibt es einen Zweistufen-Komprimierungsplan. In der ersten Stufe wird die Lauflängenkodierung (RLE) und in der zweiten Stufe die anpassungsfähige Prefixkodierung (adaptive prefix coding) angewendet.

In bekannten technischen Lösungen kommt die Informationsverarbeitung in zwei Phasen zustande: Zuerst wird die Information nach dem RLE-Verfahren bearbeitet. Danach wird die resultierende Kode-Sequenz unter Einsatz von Prefix-Kodierung "nachbearbeitet".

Anhand von zahlreichen Studien wurde nachgewiesen, daß der Hauptinformationsträger sowohl innerhalb des biologischen Objektes als auch unter einzelnen biologischen Objekten, darunter auch unter Menschen, die Energie-Informationsstrahlung von feinen physischen Feldern (fine physical fields - FPF) ist. Der eigentliche Informationsübertragungsvorgang ist ein Energie-, räumlicher und zeitlicher Vorgang. Die Studien haben gezeigt, daß die Raum-Zeit-Struktur der Außen- Makrowelt eine ununterbrochen wiederkehrende Reihe von Einwirkungen durchmachte und dadurch in ein chemisches Kontinuum der molekulären Mikrowelt der Lebewesen umgewandet wurde. Dabei trug sie der Umwandlung der chemischen Gefüge in die Funktionsstrukturen bei. Der lebendige und der leblose Stoff bestehen aus den gleichen Elementen des Periodensystems. Bei der Abgrenzung von lebendigem und leblosem Stoff spielt der Charakter der Struktur eine wichtige Rolle, allerdings nur wenn die jeweilige Struktur eine bestimmte Funktion hat. Der Übergang von Leben zu Tod unter Beibehaltung der vorhandenen Struktur und Abbruch der lebensspezifischen (das Leben festlegenden) Funktionen ist nur für lebendigen Stoff möglich. Die allgemeine Beschaffenheit des Lebendigen ist das Reproduktionsvermögen. Jedoch besteht die kennzeichnende Haupteigenschaft darin, dass jedes Lebewesen seine eigene individuelle Informations-Befehls-Struktur hat, denn die Selbstreproduktion ohne Vererblichkeit von Information und Entwicklungsabläufe unmöglich ist.

Dieser Gruppe von Erfindungen liegt Folgendes zugrunde.
Allen Substanzen und Gegenständen unserer Umwelt einschließtich der biologischen Objekte ist eine gewisse sehr schwache Strahlung eigen. Diese Strahlungen sind von der Natur her Informationswellen und enthalten Informationen über die Eigenschaften des strahlenden Objektes (Strahlungsquelle). Sie werden unter die so genannten feinen physischen Felder eingereiht. Zu den Eigenschaften der Energie-Infonnationsstrahlung der feinen physischen Felder zählt das Phasenvariationsvermögen sogar bis zu Inversion, wenn diese Strahlung über den optischen Weg läuft. Bei elektromagnetischer Strahlung kann solcher Effekt nur im optischen Spektralbereich beobachtet werden. Im Gegenteil, bei feinen physischen Feldern hängt das Phasenvariationsvermögen mit ihren Frequenzkenndaten nicht zusammen. Dabei besteht die Gleichartigkeit des polarisierten Lichts und der Strahlung von den Quellen der feinen physischen Felder darin, daß die Energie-Informationsstrahlungen ebenfalls linear polarisierte Schwingungen darstellen, und dass in beiden Strahlungen das Vorhandensein von Linearpolarisation anhand von gleichen Analysengeräten erkannt werden kann. Es wurde festgestellt, daß eine der Eigenschaften der feinen physischen Felder darin besteht, daß sie unter bestimmten Bedingungen mit den Objekten der Umwelt und besonders mit biologischen Objekten z.B. mit dem menschlichen Körper zusammenwirken können. Diese Zusammenwirkung zeigt sich bei der Realisierung des Vorgangs der informationswellengestützten Übertragung von Eigenschaften der Quellen der feinen physischen Felder auf die Zwischen- oder End-Informationsträger. Dabei wird die Wellennachbildung eines einzelnen Teils der Quelle der feinen physischen Felder zum Träger von Informationswellenmerkmalen des gesamten Gegenstandes. Das heißt: Jeder beliebig kleiner Teil eines Trägers von feinen physischen Feldern wird die gleichen Eigenschaften haben wie die ursprüngliche Quelle. Deswegen hängt die Strahlungsintensität der flachen Quellen oder der FPF-Träger von den Abmessungen (von der Größe) der strahlenden Fläche nicht ab. Die Strahlungspolarisation ist die Ausrichtung der Wellenkraftvektoren in der Ebene, die normal zum Lichtstrahl liegt. Der Polarisator (Polarisationsfilter) ist ein Lichtfilter, welcher das nicht polarisierte oder teilweise polarisierte Licht in das linearpolarisierte Licht umsetzt. Zur Informationsübertragung und -empfang werden jeweils verschiedene Sende- und Empfangseinrichtungen eingesetzt. Dazu zählen die allgemein bekannten Telefongeräte, Fernseher und PCs.

Die technische Aufgabe der Gruppe von mit einer erfinderischen Idee verbundenen Erfindungen ist die Entwicklung von dem wirksamen Verfahren und Einrichtung zur Fernübertragung der spezifischen Energie-Informationsstrahlung.

Die gestellte Aufgabe wird dank dem erreichten technischen Ergebnis gelöst. Beim genannten technischen Ergebnis handelt es sich um:
- Sicherstellung von einer wirksamen Fernübertragung der Energie-Informationssignale,
- Vereinfachung des Arbeitsablaufs im Zusammenhang mit der Übertragung und mit dem Auftragen der mittels Energie-Informationsstrahlung gesendeten Information auf einen Fernträger,
- Verkürzung der Prozedurdauer und Erweiterung von Funktionsmöglichkeiten während der Diagnosestellung bei verschiedenen Pathologien und während der Bestimmung von erfolgsreichen Behandlungsplänen bei Anwendung in der Medizin.

Wesen der Erfindung in Bezug auf das Verfahren:
Das Verfahren zur Übertragung der Energie-Informationssignale sieht die Übertragung dieser Signale auf den ersten Zwischenträger vor, mit nachfolgender Informationsübertragung zum Eingang der Empfangseinrichtung und Ableitung dieses Signals an der Empfangsseite indem das Signal auf den zweiten Träger übertragen wird, um es bei beliebiger Anwendung bzw. im beliebigen Anwendungsgebiet benutzen zu können.

Gemäß einzelnen Ausgestaltungen des Verfahrens zur Fernübertragung des Energie-Informationssignals von lebendigen und leblosen Objekten wird das Energie-Informationssignal vom Ausgangsobjekt in das Informationswellenbündel umgewandelt und in den kohärenten Zustand gebracht, indem es über wenigstens einen zwischen zwei Polarisatoren angeordneten Kristall durchgelassen wird. Danach wird das umgewandelte Signal auf den ersten Zwischenträger aufgezeichnet. Dann wird das Signal in die Empfangs- und Sendeeinrichtung eingeleitet und über die drahtgebundenen und drahtlosen Verbindungsnetze (Verbindungsleitungen) zusammen mit dem elektromagnetischen Signal übertragen. Anschließend wird das empfangene Signal auf den zweiten Träger gespeichert, und das dem Ausgangsobjekt inhärente (eigene) Energie-Informationssignal wird vom zweiten Träger abgelesen.

Gemäß einzelnen Ausgestaltungen des Verfahrens wird das umgewandelte Signal auf den ersten Zwischenträger aufgezeichnet, indem die Strahlungen von genormten Sende- und Empfangsanlagen mithilfe von dem Energie-Informationssignal moduliert / demoduliert (ausgesteuert) werden.

Gemäß einzelnen Ausgestaltungen des Verfahrens erfolgt das Ablesen des dem Ausgangsobjekt inhärenten Energie-Informationssignals vom zweiten Träger mittels des biophysikalischen Geräts für Bioresonaztherapie oder vegetativen Resonanztest.

Gemäß einzelnen Ausgestaltungen des Verfahrens wird als Ausgangsobjekt eine Substanz aus folgender Gruppe verwendet: histologisches Präparat auf dem Objektglas, Vollblut, Blutplasma oder biologische Flüssigkeit aus folgender Gruppe: Harn, Speichel, Sperma, Träne, Schweiß, Hautabdruck und /oder ein Stück von Resektat oder sein Abdruck auf einem beliebigen Material.

Gemäß einzelnen Ausgestaltungen des Verfahrens wird zur Übertragung des Energie-Informationssignals über die drahtgebundenen und drahtlosen Verbindungsnetze (Verbindungsleitungen) die genormte Empfangs- und Sendegerätetechnik aus folgender Gruppe angewendet: Funksprechgerät, Computer, Kabel- und darunter Kupfer- und / oder Glasfaserverbindungsleitungen.

Gemäß einzelnen Ausgestaltungen des Verfahrens wird die Übertragung des Energie-Informationssignals über das Internet durchgeführt.

Gemäß einzelnen Ausgestaltungen des Verfahrens werden als Zwischenträger Optoplatte (CDs) verwendet.

Gemäß einzelnen Ausgestaltungen des Verfahrens erfolgt die Übertragung des Energie-Informationssignals der leblosen Objekte mittels Übertragung dieser Signale auf einen Zwischenträger, z.B. eine Optoplatte (CD) mit nachfolgender Informationsübertragung zum Eingang einer genormten Einrichtung, z.B. Computer, Funksprechgerät, Telefonvermittlungsstelle, und Ableitung dieses Signals an der Empfangsseite mittels Übertragung dieses Signals auf den zweiten (End-) Träger, um es nachfolgend für beliebige Anwendungen einsetzen zu können.

### Wesen der Erfindung in Bezug auf Einrichtung

Der Energie-Informationssignalwandler enthält wenigstens einen zwischen zwei Polarisatoren angeordneten Kristall. Dabei befindet sich einer der Polarisatoren an der Seite der Quelle des Energie-Informationssignals und der andere Polarisator ist an der Seite des durch das umgewandelte Signal beanspruchten Objektes angeordnet.

Gemäß einzelnen Ausgestaltungen der Einrichtung wird wenigstens ein Kristall zwischen zwei gekreuzten (und /oder parallel liegenden) Polarisatoren angeordnet.

Gemäß einzelnen Ausgestaltungen der Einrichtung ist der Wandler mit einer Auflagefläche für das Objekt versehen, welches die Quelle des superschwachen Energie-Informationssignals darstellt. Dabei wird die Auflagefläche unter einem Polarisator angeordnet. Der Behälter für den Zwischenträger wird oberhalb des anderen Polarisators angeordnet.

Gemäß einzelnen Ausgestaltungen der Einrichtung ist der Behälter mit einem Kabel zum Anschluß an den Stromkreis des biophysikalischen für die Bioresonaztherapie oder den vegetativen Resonanztest geeigneten Geräts ausgestattet.

Gemäß einzelnen Ausgestaltungen der Einrichtung enthält der Wandler wenigstens einen Kristall aus folgender Gruppe: Quarzkristalle, Doppelspatkristalle, Flüssigkristalle.

Gemäß einzelnen Ausgestaltungen der Einrichtung ist einer der Polarisatoren fest (ortsgebunden), und der andere drehbar aufgestellt, so daß der Drehwinkel bis 90° Grad ermittelt werden kann.

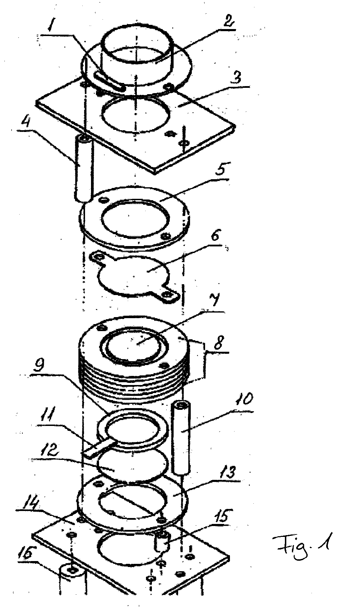

Die Zeichnung aus Fig. 1 enthält ein Beispiel für die Ausführung des Wandlers.

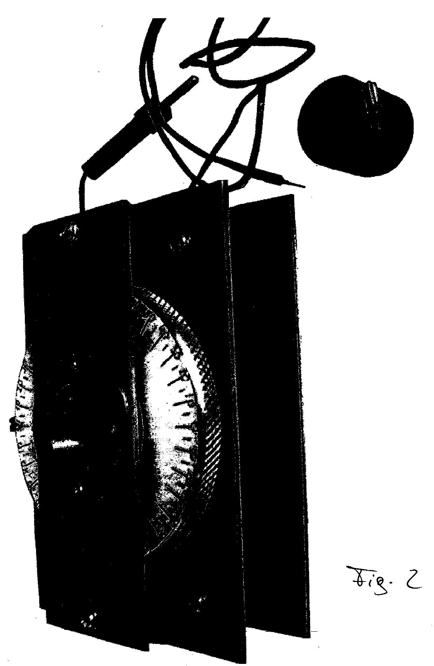

Fig. 2 zeigt die Gesamtansicht des Wandlers in der axonometrischen Projektion.

Die Bauteile in Fig. 1 sind folgerichtig getrennt dargestellt, und zwar gemäß der Signalrichtung von unten nach oben.
Der Wandler enthält Kontakt 1, Becher (Behälter) 2 für die Aufnahme des Objekts zur Einwirkung (Beanspruchung) durch superschwaches Energie-Informationssignal, obere Grundplatte 3, die erste Stütze 4, Folienhalter 5, obere Polarisationsfolie 6 (Polarisator), Quarzplatte 7, Quarzhalter 8 (6 Stck.), den zweiten Folienhalter 9, die zweite Stütze 10, Justiermitnehmer 11, untere Polarisationsfolie 12 (Polarisator), unteren Ring 13 für Folienhalter 12, untere Grundplatte 14, die dritte Stütze 15, Dämpfer 16 (4 Stck.), eine Auflagefläche (nicht abgebildet) für die Quelle des Energie-Informationssignals.

Kontakt 1 dient zur Verbindung mit einem Gerät für vegetativen Resonanztest, wenn der Wandler in Kombination mit diesem Gerät betrieben wird. Die Verbindung wird anhand des Kabels hergestellt. Becher 2 nimmt einen Behälter mit einer Substanz (homöopathisches Granulat, Alkohol, Wasser usw.) auf. Diese Substanz dient zur "Aufzeichnung" (Speicherung) der verstärkten und (mit Hilfe von einem System der Polarisatoren und Kristalle) umgewandelten Information vom Ausgangspräparat. Das Ausgangspräparat befindet sich dabei in einer Bohrung in der unteren Grundplatte 14 in der Auflagefläche. Obere Grundplatte 3 dient zur Befestigung des Bechers 2. Stützen 4, 10, 15 (oben, mittig und unten) binden die gesamte Konstruktion der Einrichtung zusammen und sorgen für die baumäßigen Spiele zwischen Kristall 7 und Polarisatoren 6, 12. Quarzhalter 8 (6 Stck.) sind mit runden Bohrungen für die Aufnahme der Quarzplatten 7 versehen. Jede Quarzplatte 7 wird in einem separaten Halter 8 festgemacht. Untere Polarisationsfolie 12 wird am Ring 13 des unteren Halters fixiert. Der untere Halter wird drehbar mit Mitnehmer 11 aufgebaut. Justiermitnehmer 11 sorgt für die Schwenkung des Polarisators 12, bis das maximale Signal am Kontakt 1 erreicht ist. Polarisatoren 6, 12 müssen parallel sein. Sie können in einer beliebigen Ebene von horizontal bis vertikal liegen. Dabei ist der untere Ring 13 für Folienhalter 12 (Polarisator) dafür erforderlich, um den ersten (unteren) Polarisator 12 zu unterstützen, welcher am Kreisumfang in der horizontalen oder anderen Ebene bewegt wird. In der Mitte der unteren Grundplatte 14 ist ein Rundloch ausgebildet, um darin die Auflagefläche (Schale) mit der Ausgangssubstanz (Präparat) zu platzieren. Die Ausgangssubstanz (Präparat) ist die Quelle des Energie-Informationssignals. Dämpfer 16 (4 Füße) stellen den Schutz gegen Außenvibrationen und sonstige mechanische Beanspruchungen sicher.

Das Energie-Informationssignal verläuft durch eine Linse, die gekreuzten Polarisatoren und/oder Kristall des Verstärkers - des Wandlers und verstärkt das vom lebendigen und leblosen Stoff kommende Informationssignal. Das sowohl von lebendigen als auch von leblosen Objekten kommende Signal hat ebenfalls solche Eigenschaften wie Fokussierung (Kohärenz), Polarisation und Verstärkung. Die gesammelten Ergebnisse haben gezeigt, daß das Informationssignal dank der Fokussierung verstärkt und in den kohärenten Zustand gebracht werden kann. Die Fokussierung erfolgt über die Bikonvexlinsen wie es beim normalen Licht im sichtbaren Bereich der Fall ist. Die Fokussierung erfolgt auch über Polarisatoren und Kristalle, wo die überflüssige Hintergrundinformation (Lastinformation) "abgeschnitten" wird.

Das vom biologischen Objekt abgeleitete Informationsfeld wird auf den ersten Träger mit Hilfe von einem Magnetinduktor oder nach einem anderen allgemein anerkannten Verfahren aufgezeichnet. Der zweite Träger wird auf die Auflagefläche unter den Polarisator aufgestellt. Das Signal läuft durch den Wandler (nachfolgend auch bedeutungsgleich Verstärker genannt) und kommt in die Ableitungseinrichtung ein. Dann wird es über Kabel und MT-Anschluß (medikamentöses Testen) an das Gerät für vegetativen Resonanztest gesendet.

Bei der Ausführung des Verfahrens kommt die Übertragung solcher Energie-Informationssignale auf den ersten Zwischenträger mit nachfolgender Informationsübertragung an den Eingang der Empfangseinrichtung und Ableitung dieses Signals an der Empfangsseite zustande, indem das Signal auf den zweiten Zwischenträger (Endträger) übertragen wird, um es nachher bei beliebiger Anwendung für beliebige Zwecke benutzen zu können. Um die Fernübertragung des Energie-Informationssignals von lebendigen und leblosen Objekten sicherzustellen, wird das Energie-Informationssignal vom Ausgangsobjekt in das Informationswellenbündel umgewandelt und in den kohärenten Zustand gebracht, indem es über wenigstens einen zwischen zwei Polarisatoren angeordneten Kristall durchgelassen wird. Danach wird das umgewandelte Signal auf den ersten Zwischenträger aufgezeichnet. Dann wird das Signal in die Empfangs- und Sendeeinrichtung eingeleitet und über die drahtgebundenen und drahtlosen Verbindungsnetze (Verbindungsleitungen) zusammen mit dem elektromagnetischen weherigestützten Signal übertragen. Anschließend wird das empfangene Signal auf den zweiten Träger gespeichert, und das dem Ausgangsobjekt eigene Energie-Informationssignal wird vom zweiten Träger abgelesen. Die Aufzeichnung des umgewandelten Signals auf den ersten Zwischenträger wird mittels Modulation / Demodulation der Strahlungen von genormten Sende- und Empfangsanlagen mithilfe von dem Energie-Informationssignal vorgenommen. Das Ablesen des dem Ausgangsobjekt inhärenten Energie-Informationssignals vom zweiten Träger erfolgt unter Einsatz des biophysikalischen Geräts für Bioresonaztherapie oder vegetativen Resonanztest. Als Ausgangsobjekt wird eine Substanz aus folgender Gruppe verwendet: histologisches Präparat auf dem Objektglas, Vollblut, Blutplasma oder biologische Flüssigkeit aus folgender Gruppe: Harn, Speichel, Sperma, Träne, Schweiß, Hautabdruck und /oder ein Stück von Resektat oder sein Abdruck auf einem beliebigen Material. Zur Übertragung des Energie-Informationssignals über die drahtgebundenen und drahtlosen Verbindungsnetze (Verbindungsleitungen) wird die standardisierte (einheitliche) Empfangs- und Sendegerätetechnik aus folgender Gruppe verwendet: Funksprechgerät, Computer, Kabel- und darunter Kupfer- und / oder Glasfaserverbindungsleitungen. Die Übertragung des Energie-Informationssignals erfolgt z.B. über Internet. Als Zwischenträger werden Optoplatten (CDs) verwendet. Somit erfolgt die Übertragung des Energie-Informationssignals der leblosen Objekte gemäß der bevorzugten Ausgestaltung des Verfahrens mittels Übertragung dieser Signale auf einen Zwischenträger, z.B. eine Optoplatte (CD) mit nachfolgender Informationsübertragung zum Eingang einer genormten Einrichtung, z.B. Computer, Funksprechgerät, Telefonvermittlungsstelle, und mittels Ableitung dieses Signals an der Empfangsseite, indem es auf den Endträger übertragen wird, um es nachfolgend für beliebige Anwendungen einsetzen zu können.

Die gewerbliche Anwendbarkeit des angemeldeten Verfahrens wird anhand der Beispiele nachgewiesen. Bei diesen Beispielen handelt es sich um Diagnosestellung. Das Informationsfeld wurde von einem Träger in Form von biologischer Flüssigkeit (Harn, Blut, Speichel) oder von den für histologische Analyse verwendeten Objektgläsern abgeleitet, umgewandelt und nachher an das Gerät für vegetativen Resonanztest gesendet (nachfolgend Transfertest genannt). Die Ergebnisse der Transfertests wurden mit den Ergebnissen der klinischen Untersuchungen verglichen. Bei klinischen Untersuchungen handelt es sich dabei um vielseitige andauernde Untersuchungen des Patienten und Studien der in der Krankengeschichte angeführten Daten. Die Untersuchungen wurden im Zeitraum August - Oktober 2009 durchgeführt.

### Beispiel 1

Mann, 58 Jahre.
Die Information wurde von der biologischen Flüssigkeit (Harn) abgelesen.
Ergebnisse von Transfertest.
Fettleber. Neigung zur Hypertonie. Erhöhter Cholesterinspiegel.
Adenomatöse Prostatahypertrophie.
Lendenwirbel.
Klinisch gestellte Diagnose (Auszug aus der Krankengeschichte):
1. Insult,
2. Infarkt,
3. Podagra,
4. Hochdruckkrankheit 2. St.
5. Diszirkulatorische Hochdruck- und Post-Insultenzephalopathie 2. St.
6. Cholelithiasis, chr. Gallensteinleiden in der Remissionsphase.
7. Echo-Merkmale von diffusen Änderungen in der Leber, chr. Uratendiathese, Nierenzyste links.

### Beispiel 2

Frau, 54 Jahre.
Die Information wurde von der biologischen Flüssigkeit abgelesen (Harn).
Ergebnisse von Transfertest.
Chronische Adnexitis. Eileiter. Parametralraum. Schwarten in der Bauchhöhle und im kleinen Becken. Myom. Herpes 2. Psychovegetative Belastungen.
Streßbelastung 5. St. Endokrinstörungen. Harnsäuresalze.
Rheumatismus in der Remissionsphase. Hämolytischer Streptokokkus. Lendenwirbel.
Klinisch gestellte Diagnose (Auszug aus der Krankengeschichte):
1. Knotiger Kropf
2. Stoffwechselstörung,
3. Gonarthrose
4. Chr. Cholezystitis. Chr. sekundäre Pankreatitis
5. Mäßige Myokard-Änderungen
6. Entfernte Uterus-Myom, Endometriose
7. Degenerative dystrophische Veränderungen der Lendenwirbelsäule, Schmorls Hernie, paramediane Hernie.
8. Herpes

### Beispiel 3

Anonymer Patient.
Testgegenstand: Krebsforschung. Die Information wurde von Objektträgern abgelesen.
Ergebnis von Transfertest: Vorhandene onkologische Krankheit. Klinische Diagnose: Minderwertig differenziertes Adenokarzinom.

### Beispiel 3

Anonymer Patient. Testgegenstand: Krebsforschung. Die Information wurde von Objektträgern abgelesen.
Ergebnis von Transfertest: Keine onkologische Krankheit. Klinische Diagnose: Gesundes Gewebe.

### Beispiel 4

Anonymer Patient. Testgegenstand: Krebsforschung. Die Information wurde von Objektträgern abgelesen.
Ergebnis von Transfertest: Keine onkologische Krankheit. Klinische Diagnose: Chronische Kolitis.

### Beispiel 5

Anonymer Patient. Testgegenstand: Krebsforschung. Die Information wurde von Objektträgern abgelesen.
Ergebnis von Transfertest: Keine onkologische Krankheit. Klinische Diagnose: Granulationsgewebe.

### Beispiel 6

Anonymer Patient. Testgegenstand Krebsforschung. Die Information wurde von Objektträgern abgelesen.
Ergebnis von Transfertest: Vorhandene onkologische Krankheit. Klinische Diagnose: Bindegewebe.

### Beispiel 7

Anonymer Patient. Testgegenstand: Krebsforschung. Die Information wurde von Objektträgern abgelesen.
Ergebnis von Transfertest: Vorhandene onkologische Krankheit. Klinische Diagnose: Eierstockkrebs.

### Beispiel 8

Anonymer Patient. Testgegenstand Krebsforschung. Die Information wurde von Objektträgern abgelesen.
Ergebnis von Transfertest: Vorhandene onkologische Krankheit. Klinische Diagnose: Nierenkrebs.

### Beispiel 9

Anonymer Patient. Testgegenstand: Krebsforschung. Die Information wurde von Objektträgern abgelesen.
Ergebnis von Transfertest: Vorhandene onkologische Krankheit. Klinische Diagnose: Minderwertig differenziertes Adenokarzinom.

### Beispiel 10

Anonymer Patient. Testgegenstand: Krebsforschung. Die Information wurde von Objektträgern abgelesen.
Ergebnis von Transfertest: Vorhandene onkologische Krankheit. Klinische Diagnose: Leberkrebs.

### Beispiel 11

Anonymer Patient. Testgegenstand: Krebsforschung. Die Information wurde von Objektträgern abgelesen.
Ergebnis von Transfertest: Vorhandene onkologische Krankheit. Klinische Diagnose: Nierenkrebs.

### Beispiel 12

Frau, 58 Jahre.
Testgegenstand: Krebsforschung.
Die Information wurde von der biologischen Flüssigkeit (Harn) abgelesen.
Ergebnis von Transfertest: Keine onkologische Krankheit. Klinische Diagnose: Keine onkologische Krankheit.

### Beispiel 13

Frau, 54 J.
Testgegenstand: Krebsforschung.
Die Information wurde von der biologischen Flüssigkeit (Harn) abgelesen.
Ergebnis von Transfertest: Keine onkologische Krankheit. Klinische Diagnose: Keine onkologische Krankheit.

### Beispiel 14

Frau, 64 J.
Testgegenstand: Krebsforschung.
Die Information wurde von der biologischen Flüssigkeit (Harn) abgelesen.
Ergebnis von Transfertest: Keine onkologische Krankheit Klinische Diagnose: Keine onkologische Krankheit.

### Beispiel 15

Mann, 54 Jahre.
Testgegenstand: Krebsforschung. Die Information wurde von der biologischen Flüssigkeit (Harn) abgelesen.
Ergebnis von Transfertest: Vorhandene onkologische Krankheit. Klinische Diagnose: Vorhandene onkologische Krankheit.

### Beispiel 16

Mann, 54 J.
Testgegenstand: Krebsforschung.
Die Information wurde von der biologischen Flüssigkeit (Harn) abgelesen.
Ergebnis von Transfertest: Keine onkologische Krankheit. Klinische Diagnose: Vorhandene onkologische Krankheit.

### Beispiel 17

Frau, 76 Jahre.
Testgegenstand: Magengeschwür.
Die Information wurde von der biologischen Flüssigkeit (Harn) abgelesen.
Ergebnis von Transfertest: Kein Magengeschwür. Klinische Diagnose: Kein Magengeschwür.

### Beispiel 18

Frau, 23 J.
Testgegenstand: Magengeschwür.
Die Information wurde von der biologischen Flüssigkeit (Harn) abgelesen.
Ergebnis von Transfertest: Magengeschwür vorhanden. Klinische Diagnose: Magengeschwür vorhanden.

### Beispiel 19

Frau, 24 J.
Testgegenstand: Magengeschwür.
Die Information wurde von der biologischen Flüssigkeit (Harn) abgelesen.
Ergebnis von Transfertest: Kein Magengeschwür. Klinische Diagnose: Magengeschwür vorhanden.

### Beispiel 20

Frau, 50 Jahre.
Testgegenstand: Magengeschwür.
Die Information wurde von der biologischen Flüssigkeit (Harn) abgelesen.
Ergebnis von Transfertest: Kein Magengeschwür. Klinische Diagnose: Kein Magengeschwür.

### Beispiel 21

Mann, 33 J.
Testgegenstand: Magengeschwür.
Die Information wurde von der biologischen Flüssigkeit (Blut) abgelesen.
Ergebnis von Transfertest: Magengeschwür vorhanden. Klinische Diagnose: Magengeschwür vorhanden.

### Beispiel 22

Mann, 26 Jahre.
Testgegenstand: Hepatitis. Die Information wurde von der biologischen Flüssigkeit (Harn) abgelesen.
Ergebnis von Transfertest: Vorhandene Hepatitis. Klinische Diagnose: Vorhandene Hepatitis.

### Beispiel 23

Mann, 34 J.
Testgegenstand: Hepatitis. Die Information wurde von der biologischen Flüssigkeit (Harn) abgelesen.
Ergebnis von Transfertest: Vorhandene Hepatitis. Klinische Diagnose: Vorhandene Hepatitis.

### Beispiel 24

Frau, 54 J.
Testgegenstand: Hepatitis. Die Information wurde von der biologischen Flüssigkeit (Speichel) abgelesen.
Ergebnis von Transfertest: Keine Hepatitis vorhanden. Klinische Diagnose: Keine Hepatitis vorhanden.

### Beispiel 25

Frau, 48 Jahre.
Testgegenstand: Hepatitis. Die Information wurde von der biologischen Flüssigkeit (Harn) abgelesen.
Ergebnis von Transfertest: Vorhandene Hepatitis. Klinische Diagnose: Vorhandene Hepatitis.

Somit bestätigen die Ergebnisse der durchgeführten und oben aufgelisteten Tests die hohe Genauigkeit bei der Diagnosestellung, welche in einem Fernmodus und sehr kurzfristig vorgenommen wurde. Folglich wurde festgestellt und nachgewiesen, daß die Übertragung, der Empfang und das Ablesen des Energie-Informationssignals vom Ausgangsobjekt vorhanden und möglich ist.

Somit wurden das wirksame Verfahren und Einrichtung zur Fernübertragung der spezifischen Energie-Informationsstrahlung entwickelt.

Dabei wurde wirksame Fernübertragung der Energie-Informationssignale sichergestellt. Der Arbeitsablauf im Zusammenhang mit der Übertragung und mit dem Auftragen der mit der Energie-Informationsstrahlung gesendeten Information auf den Fernträger wurde vereinfacht. Darüber hinaus wird die Prozedurdauer bei der Anwendung der Erfindung in der Medizin verkürzt, und die Funktionsmöglichkeiten für die Erkennung von verschiedenen Pathologien bei Diagnosestellung und für die Bestimmung von erfolgsreichen Behandlungsplänen wurden erweitert.

## Patentansprüche

1. Verfahren zur Übertragung von Energie-Informationssignalen, welches die Übertragung dieser Signale auf den ersten Zwischenträger mit nachfolgender Informationsübertragung zum Eingang der Empfangseinrichtung und Ableitung dieses Signals an der Empfangsseite vorsieht, indem das Signal auf den zweiten Träger übertragen wird, um es bei beliebiger Anwendung bzw. im beliebigen Anwendungsgebiet benutzen zu können.

2. Verfahren nach Anspruch 1,
**dadurch gekennzeichnet,**
**daß** das Energie-Informationssignal vom Ausgangsobjekt zur Fernübertragung des Energie-Informationssignals von lebendigen und leblosen Objekten in das Informationswellenbündel umgewandelt und in den kohärenten Zustand gebracht wird, indem es über wenigstens einen zwischen zwei Polarisatoren angeordneten Kristall durchgelassen wird; danach wird das umgewandelte Signal auf den ersten Zwischenträger aufgezeichnet und in die Empfangs- und Sendeeinrichtung eingeleitet, anschließend über die drahtgebundenen und drahtlosen Verbindungsnetze (Verbindungsleitungen) zusammen mit dem elektromagnetischen Signal übertragen; danach wird das empfangene Signal auf den zweiten Träger gespeichert, und das dem Ausgangsobjekt inhärente Energie-Informationssignal wird vom zweiten Träger abgelesen.

3. Verfahren nach Anspruch 2, **dadurch gekennzeichnet, daß** das umgewandelte Signal auf den ersten Zwischenträger aufgezeichnet wird, indem die Strahlungen von genormten Sende- und Empfangsanlagen mithilfe von dem Energie-Informationssignal moduliert / demoduliert werden.

4. Verfahren nach Anspruch 2, **dadurch gekennzeichnet, daß** das Ablesen des dem Ausgangsobjekt inhärenten Energie-Informationssignals vom zweiten Träger unter Einsatz des biophysikalischen Geräts für Bioresonaztherapie oder vegetativen Resonanztest erfolgt.

5. Verfahren nach einem der Ansprüche 1 - 4, **dadurch gekennzeichnet, daß** als Ausgangsobjekt eine Substanz aus folgender Gruppe verwendet wird: histologisches Präparat auf dem Objektglas, Vollblut, Blutplasma oder biologische Flüssigkeit aus folgender Gruppe: Harn, Speichel, Sperma, Träne, Schweiß, Hautabdruck und /oder ein Stück von Resektat oder sein Abdruck auf einem beliebigen Material.

6. Verfahren nach einem der Ansprüche1 - 4, **dadurch gekennzeichnet, daß** zur Übertragung des Energie-Informationssignals über die drahtgebundenen und drahtlosen Verbindungsnetze (Verbindungsleitungen) genormte Empfangs- und Sendegerätetechnik aus folgender Gruppe eingesetzt wird: Funksprechgerät, Computer, Kabel- und darunter Kupfer- und / oder Glasfaserverbindungsleftungen.

7. Verfahren nach einem der Ansprüche1 - 4, **dadurch gekennzeichnet, daß** die Übertragung des Energie-Informationssignals über das Internet durchgeführt wird.

8. Verfahren nach einem der Ansprüche1 - 4, **dadurch gekennzeichnet, daß** als Zwischenträger Optoplatten (CDs) verwendet werden.

9. Verfahren nach einem der Ansprüche 1 - 4, **dadurch gekennzeichnet, daß** die Übertragung des Energie-Informationssignals der leblosen Objekte mittels Übertragung dieser Signale auf einen Zwischenträger, z.B. eine Optoplatte (CD) mit nachfolgender Informationsübertragung zum Eingang einer genormten Einrichtung, z.B., Computer, Funksprechgerät, Telefonvermittlungsstelle, und Ableitung dieses Signals an der Empfangsseite erfolgt, indem das Signal auf den zweiten Träger übertragen wird, um es in einem beliebigen Anwendungsbereich benutzen zu können.

10. Energie-Informationssignalwandler mit wenigstens einem Kristall, welcher zwischen zwei Polarisatoren angeordnet wird; dabei befindet sich einer der Polarisatoren an der Seite der Quelle des Energie-Informationssignals und der andere Polarisator ist an der Seite des durch das umgewandelte Signal beanspruchten Objektes angeordnet.

11. Wandler nach Anspruch 10, **dadurch gekennzeichnet, daß** wenigstens ein Kristall zwischen zwei gekreuzten (und /oder parallel liegenden) Polarisatoren angeordnet ist.

12. Wandler nach Anspruch 11, **dadurch gekennzeichnet, daß** er mit einer Auflagefläche für das Objekt und einem Behälter für den Zwischenträger versehen ist, wobei das Objekt die Quelle des superschwachen Energie-Informationssignals darstellt, und daß die Auflagefläche unter einem Polarisator und der Behälter für den Zwischenträger oberhalb des anderen Polarisators angeordnet wird.

13. Wandler nach Anspruch 11, **dadurch gekennzeichnet, daß** der Behälter mit einem Kabel für den Anschluß an den Stromkreis des für die Bioresonaztherapie oder den vegetativen Resonanztest geeigneten biophysikalischen Geräts versehen ist.

14. Wandler nach einem der Ansprüche 10 - 13, **dadurch gekennzeichnet, daß** er wenigstens einen Kristall aus folgender Gruppe enthält: Quarzkristalle, Doppelspatkristalle, Flüssigkristalle.

15. Wandler nach einem der Ansprüche 10 - 13, **dadurch gekennzeichnet, daß** einer der Polarisatoren fest und der andere Polarisator drehbar aufgestellt ist, so daß der Drehwinkel bis 90° Grad ermittelt werden kann.
